Europäisches Patentamt

European Patent Office

Office européen des brevets

(19)

(11) Publication number: **0 040 489**

**A1**

(12)

# EUROPEAN PATENT APPLICATION

(21) Application number: **81301980.9**

(22) Date of filing: **06.05.81**

(51) Int. Cl.³: **A 61 K 45/06**
**A 61 K 31/415, A 61 K 31/505**
**A 61 K 31/35**
**//(A61K31/415, 31/35),**
**(A61K31/505, 31/35), (A61K31/35,**
**31/34)**

---

(30) Priority: **17.05.80 GB 8016381**

(43) Date of publication of application:
**25.11.81 Bulletin 81/47**

(84) Designated Contracting States:
**AT BE CH DE FR GB IT LI LU NL SE**

(71) Applicant: **FISONS LIMITED**
**Fison House 9 Grosvenor Street**
**London WIX OAH(GB)**

(72) Inventor: **Wilson, Jeffrey**
**5 Pantain Road**
**Loughborough Leicestershire(GB)**

(72) Inventor: **Lee, Thomas Brian**
**51 Outwoods Road**
**Loughborough Leicestershire(GB)**

(74) Representative: **Craig, Christopher Bradberry et al,**
**Fisons Limited Fison House Princes Street**
**Ipswich 1P1 1QH(GB)**

---

(54) Mixtures, salts, packages and pharmaceutical compositions containing
5-(2-hydroxypropoxy)-8-propyl-4H-1-benzopyran-2-carboxylic acid or a derivative thereof and an H2 receptor antagonist
antihistamine.

(57) There is described a pharmaceutical mixture comprising
(a) one or more of 5- (2-hydroxypropoxy)-8-propyl-4H-1-
benzopyran-2-carboxylic acid, or a pharmaceutically accept-
able derivative thereof, in admixture with
(b) one or more antihistamines having $H_2$ receptor antagon-
ist properties.

Also described are salts of 5-(2-hydroxypropoxy)-8-
propyl-4H-1-benzopyran-2-carboxylic acid with an antihis-
tamine having $H_2$ receptor antagonist properties.

EP 0 040 489 A1

MIXTURES, SALTS, PACKAGES AND PHARMACEUTICAL COMPOSITIONS CONTAINING 5-(2-HYDROXYPROPOXY)-8-PROPYL-4H-1-BENZOPYRAN-2-CARBOXYLIC ACID OR A DERIVATIVE THEREOF AND AN $H_2$ RECEPTOR ANTAGONIST ANTIHISTAMINE

This invention relates to new mixtures and methods for their preparation and use.

In British Patent Specification No 1,488,707 5-(2-hydroxypropoxy)-4-oxo-8-propyl-4H-1-benzopyran-2-carboxylic acid, and its pharmaceutically acceptable derivatives, are described as being useful in the treatment of certain conditions involving antigen-antibody reactions, and notably allergic asthma. We have now found that these compounds can inhibit gastric acid secretion and are useful in the treatment of conditions in which it is desirable to lower the level of gastric acid secretion. What is more we have found that under certain conditions, the maximal effect of these compounds can be increased by mixing them (or forming salts) with certain antihistamines having $H_2$ receptor antagonist properties. These antihistamines have some undesirable side effects, and by use of the mixtures and salts of the present invention the necessary dosage of the antihistamine can be reduced thus avoiding, or mitigating the effect of, those side effects.

According to our invention we provide a pharmaceutical mixture comprising

(a) one or more of 5-(2-hydroxypropoxy)-8-propyl-4H-1-benzopyran-2-carboxylic acid, or a pharmaceutically acceptable derivative thereof, (herein referred to collectively as 'active ingredient') in admixture with

(b) one or more antihistamines having $H_2$ receptor antagonist properties.

Pharmaceutically acceptable derivatives of 5-(2-hydroxypropoxy)-8-propyl-4H-1-benzopyran-2-carboxylic acid include pharmaceutically acceptable salts, esters and amides of the 2-carboxylic acid group. Suitable salts include ammonium, alkali metal (e.g. sodium, potassium and lithium) and alkaline earth metal salts (e.g. calcium or magnesium), and salts with suitable organic bases, e.g. salts with lower alkylamines such as methylamine or ethylamine, with substituted lower alkylamines, e.g. hydroxy substituted alkylamines or with simple monocyclic nitrogen heterocyclic compounds, e.g. piperidine or morpholine. Suitable esters include simple lower alkyl (e.g. C 1 to 10) esters, esters derived from alcohols containing basic groups, e.g. di-lower alkyl (e.g. C 1 to 10) amino substituted alkanols, and acyloxy alkyl esters, e.g. a lower acyl-lower alkyl ester, or a bis-ester derived from a di-hydroxy compound, e.g. a di(hydroxy-lower alkyl)ether. The pharmaceutically acceptable salts of the basic esters, e.g. the hydrochloride, may also be used. The esters may be made by conventional techniques, e.g. esterification or transesterification. We prefer to use the free acid 5-(2-hydroxypropoxy)-8-propyl-4H-1-benzopyran-2-carboxylic acid, or a salt, e.g. the sodium salt, thereof.

The antihistamine may be, for example, cimetidine, i.e. N-cyano-N'-methyl-N'[2-(5-methyl-4-imidazolylmethyl-thio)ethyl] guanidine, ranitidine; i.e. N-[2-[[[5-[di-methylamino)-methyl]-2-furanyl]methyl]thio]ethyl]-N'--methyl-2-nitro-1,1-ethenediamine; or oxmetidine, i.e. 5-(1,3-benzodioxol-5-yl-methyl)-2-[[2-[[(5-methyl-1H-imidazol-4-yl)-methyl]thio]ethyl]amino]-4-1(H)-pyrimidinone.  The antihistamine may be used in the form of the free base or of a pharmaceutically acceptable salt thereof, e.g. the hydrochloride.

The ratio of active ingredient and antihistamine in the composition will vary with the particular active ingredient and antihistamine used and the specific purpose for which the composition is intended.  However, we have found that in general a ratio of from 0.5 to 80, preferably from 6 to 40, and more preferably from 1 to 10 parts by weight of active ingredient (measured as the free acid) for each part by weight of antihistamine is appropriate.

We prefer the composition to contain only one active ingredient and only one antihistamine.

A suitable daily dose of active ingredient for most purposes is in the range 0.5 to 4.0g and preferably 1 to 2g, (measured as the free acid).

A suitable daily dosage of antihistamine will vary with the antihistamine, but for cimetidine, is in the range 0.2 to 1.0, and preferably 0.4 to 0.8g per day, and for ranitidine and oxmetidine is from 0.05 to 0.25, and preferably 0.1 to 0.2g per day. The dose of the antihistamine may be lowered by use in admixture with the active ingredient.

The composition may be administered as divided doses from 1 to 6, and preferably from 2 to 4, times per day. Each dose may comprise one or more unit doses.

We particularly prefer compositions containing cimetidine.

According to our invention we also provide a method for the treatment of a condition of the gastrointestinal tract, e.g. excess gastric acid secretion, peptic, duodenal or recurrent ulceration, non-ulcer dyspepsia, duodenitis, Zollinger-Ellison syndrome, reflux oesophagitis and the management of ulcers in the upper gastrointestinal tract, which comprises administration of a composition according to the invention to an individual mammal, e.g. human, suffering from such a condition. The administration is preferably oral administration.

According to the invention we also provide a method for the treatment of the above conditions of the gastrointestinal tract, which comprises sequential or simultaneous administration of active ingredient and an anti-

80/16381/4840/100          - 5 -

histamine having $H_2$ receptor antagonist properties to an individual mammal, e.g. human, suffering from such a condition.

When sequential or simultaneous administration of active ingredient and antihistamine is used the ratios and dosages of the active ingredient and antihistamine are as described above with respect to the mixtures.

The invention therefore also provides a pharmaceutical package comprising at least one unit dose of active ingredient and at least one unit dose of the antihistamine. The unit doses are preferably arranged in the package in a particular order together with written or printed indications or directions, the indications or directions and the manner of packing being such as to provide guidance in relation to and to facilitate the taking of a unit dose of active ingredient and a unit dose of the antihistamine in a particular order, e.g. a unit dose of the former before a unit dose of the latter. The package is preferably a sealed package and may comprise a tube, box or chart in or on which the unit doses are packed. The unit doses are preferably suitable for oral administration and preferably contain the doses of active ingredient and the antihistamine in the ratios set out above for the mixtures.

According to the invention we also provide a salt of 5-(2-hydroxypropoxy)-8-propyl-4H-1-benzopyran-2-carboxylic acid with those antihistamines having $H_2$ receptor antagonist properties which are sufficiently basic to form such a salt, e.g. ranitidine, cimetidine or oxmetidine.

The salt may be made by a metathetical process, e.g. by reacting a suitable salt, such as the sodium salt, of 5-(2-hydroxypropoxy)-8-propyl-4H-1-benzopyran-2-carboxylic acid with an appropriate salt, e.g. the hydrochloride or sulphate, of the antihistamine base. However the salt is preferably produced by reacting the free acid with the free antihistamine base, as such a process does not produce a bi-product inorganic salt. The reaction may be carried out in a solvent which is inert under the reaction conditions. The solvent is preferably one in which the desired salt is soluble, e.g. water. The desired salt may be isolated and purified, for example, by crystallisation or by freeze drying.

In order to produce suitable compositions the salt or the active ingredient and the antihistamine, either separately or as a mixture thereof, are worked up with organic or inorganic pharmaceutically acceptable adjuvants or excipients. Examples of such adjuvants are:-

For tablets and dragées: Binders, for example, cellulosic materials, e.g. microcrystalline cellulose and methyl cellulose; disintegrating agents, for example starches, e.g. maize starch; stabilisers, e.g. against hydrolysis of the active ingredients; flavouring agents, for example sugars such as lactose; fillers; stearates and inorganic diluents, e.g. talc.

For syrups, suspensions or dispersions: A liquid vehicle in which the active ingredients may be dissolved or suspended, e.g. water; and suspending agents, e.g. cellulose derivatives, gums etc.

For hard or soft capsules: Diluents, e.g. lactose; glidants, e.g. stearates; inorganic materials, e.g. silica or talc; stabilisers and dispersing agents.

For suppositories: Natural or hardened oils, waxes etc. A large number of proprietary emulsifying bases are available and are suitable for use in suppositories. These include 'Witepsol' bases, consisting of hydrogenated triglycerides or lauric acid with added monoglycerides; and 'Massupol' bases, which consist of glyceryl esters of lauric acid with a very small amount of glyceryl monostearate.

For enemas: Water, sodium chloride, buffers etc.

The composition may also contain further adjuvants, for example a composition for use in tablets may contain lubricants and glidants to assist in tabletting, e.g. magnesium stearate, or wetting agents to assist in granulation, e.g. dioctyl sodium sulphosuccinate. The composition may also, if desired, contain a pharmaceutically acceptable dye or colourant, and may, if desired, be coated using conventional film or sugar coating techniques.

If desired the composition, or one or more components thereof, may be formulated in sustained release form, e.g. by coating some or all of the drug particles themselves or granules thereof made with, for example, sucrose and of a size up to 2mm in diameter with a layer of, e.g. beeswax, Carnuba wax, stearic or palmitic acids, cetyl alcohol or similar substances which could be expected to be slowly dissolved or digested or to act as semi-permeable membranes through which the active ingredients can diffuse when the preparations are ingested. The composition may contain drug particles or granules which are uncoated in admixture with particles or granules having one or more coats of the coating medium, and may be in the form of a capsule containing the particles or granules or alternatively a tablet, for which other adjuvants may be required, such as glidants or lubricants. The mixture may be administered as an enteric coated composition to make the active ingredients available at the appropriate part of the gastrointestinal tract. This may be achieved by coating the tablet with a continuous film of material which is resistant and impermeable to gastric secretions, but which is susceptible to intestinal secretions.
Typical film materials are shellac and its derivatives and
cellulose acetate phthalate.

We prefer compositions which are suitable for administered oesophageally, but rectal, i.m. or i.v. administration are also envisaged.

The mixtures and compositions according to the invention may be made by mixing the active ingredient and the
antihistamine with any excipients using procedures which
are well known per se.

The invention is illustrated, but in no way limited by
the following Examples.

Example A

Tablet

| | |
|---|---|
| 5-(2-Hydroxypropoxy)-8-propyl-4H-1-benzo-pyran sodium salt | 500mg |
| Cimetidine | 200mg |
| Binder, e.g. powdered tragacanth | 1 to 3% |
| Lubricant, e.g. magnesium stearate | 0.25 to 1% |
| Disintegrating agent, e.g. potato starch | 5 to 10% |
| Surfactant, e.g. di-octylsodium sulpho-succinate | 0.25% |

80/16381/4840/100 — 10 —

Example B

Capsule (Hard)

| | |
|---|---|
| 5-(2-Hydroxypropoxy)-8-propyl-4H-1-benzo-pyran sodium salt | 500mg |
| Oxmetidine | 50mg |
| Granulating agent, e.g. gum or starch mucilage | q.s. |
| Lubricant, e.g. magnesium stearate | 0.25 to 1% |

Example C

Capsule (Soft)

| | |
|---|---|
| 5-(2-Hydroxypropoxy)-8-propyl-4H-1-benzo-pyran sodium salt | 500mg |
| Ranitidine | 50mg |
| Polyethylene glyclol 400 | q.s. |
| Non-ionic surfactant, e.g. polyoxyethylene sorbitan mono-oleate | q.s. |

Example D

Suppository

| | |
|---|---|
| 5-(2-Hydroxypropoxy)-8-propyl-4H-1-benzo-pyran sodium salt | 500mg |
| Cimetidine | 200mg |
| Basis, e.g. polyethylene glycol 6,000 | 1.4g |

Example E

The perfused stomach of the anaesthetised rat has been shown to be useful in screening drugs that inhibit gastric acid secretion.

80/16381/4840/100            - 11 -

## Materials and Methods

Male rats (200-300g) are starved for 18 hours before being anaesthetised with urethane (1.7g/kg i.p.). The trachea is cannulated. Blood pressure is recorded from a carotid artery and the jugular vein is cannulated for drug infusions and injections.

A cannula is passed down the oesophagus a pre-measured length so that the end lies near the cardiac sphincter of the stomach. This cannula is tied in place in the neck region.

A midline incision is made in the abdomen and the stomach exteriorised. The duodenum is tied off about 2cm from the pyloric sphincter. An incision is made between this tie and the sphincter and the stomach contents washed out with saline. A cannula and balloon are passed into the stomach and tied in place and the balloon is then inflated. The stomach is then replaced and the abdomen closed.

The stomach is perfused, via the oesophagus, with isotonic dextrose solution (5%), at 37°C, at a constant rate of 2ml/min. The duodenal cannula leads the effluent perfusate to a pH electrode and a continuous record of changes in pH is made throughout the experiment from which changes in hydrogen ion concentration are calculated.

When the basal acid secretion is stable, pentagastrin (4 ug/kg/hr) is infused into the jugular vein to produce an increase in gastric acid secretion. When a stable plateau is reached the test drug is injected intravenously, either by bolus injection, or, by continuous infusion.

The maximal effect of 5-(2-Hydroxypropoxy)-8-propyl-4H-1-benzopyran sodium salt (found at an infusion rate of 5mg/kg/h) was to reduce the effect of pentagastrin by 40%. This maximal effect was further increased to 55% by adding cimetidine (0.2mg/kg/hr) to the 5-(2-Hydroxy-propoxy)-8-propyl-4H-1-benzopyran sodium salt.

What we claim is:-

1. A pharmaceutical mixture comprising

(a) one or more of 5-(2-hydroxypropoxy)-8-propyl-4H-1-benzopyran-2-carboxylic acid or a pharmaceutically acceptable derivative thereof, as active ingredient, in admixture with

(b) one or more antihistamines having $H_2$ receptor antagonist properties.

2. A mixture according to Claim 1, wherein the active ingredient is 5-(2-hydroxypropoxy)-8-propyl-4H-1-benzopyran-2-carboxylic acid or a pharmaceutically acceptable salt thereof, and the antihistamine is cimetidine, ranitidine or oxmetidine.

3. A mixture according to Claim 1 or Claim 2, comprising from 0.5 to 80 parts by weight of active ingredient, measured as the free acid, for each part by weight of antihistamine.

4. A mixture according to Claim 3, comprising from 6 to 40 parts by weight of active ingredient for each part by weight of antihistamine.

5. A mixture according to Claim 4, comprising from 1 to 10 parts by weight of active ingredient for each part by weight of antihistamine.

6. A mixture according to any one of the preceding claims comprising only one active ingredient and only one antihistamine.

7. A salt of 5-(2-hydroxypropoxy)-8-propyl-4H-1-benzopyran-2-carboxylic acid with those antihistamines having $H_2$ receptor antagonist properties which are sufficiently basic to form such a salt.

8. A pharmaceutical composition comprising a mixture according to any one of Claims 1 to 6, or a salt according to Claim 7, in admixture with a pharmaceutically acceptable adjuvant or excipient.

9. A composition according to Claim 8 which is suitable for administration oesophageally.

10. A pharmaceutical package comprising at least one unit dose of active ingredient, as defined in Claim 1 and at least one dose of an antihistamine having $H_2$ receptor antagonist properties, the unit doses being arranged in the package in a particular order together with written or printed indications or directions, the indications or directions and the manner of packing being such as to provide guidance in relation to and to facilitate the taking of a unit dose of active ingredient and a unit dose of the antihistamine in a particular order.

SPECIAL CLAIMS FOR AUSTRIA

Underline: What we claim is:-

1.  A method of making a pharmaceutical mixture comprising (a) one or more of 5-(2-hydroxypropoxy)-8-propyl-4H-1-benzopyran-2-carboxylic acid or a pharmaceutically acceptable derivative thereof, as active ingredient, in admixture with

(b) one or more antihistamines having $H_2$ receptor antagonist properties,

which comprises mixing the active ingredient with the antihistamine.

2.  A method according to Claim 1, wherein the active ingredient is 5-(2-hydroxypropoxy)-8-propyl-4H-1-benzopyran-2-carboxylic acid or a pharmaceutically acceptable salt thereof, and the antihistamine is cimetidine, ranitidine or oxmetidine.

3.  A method according to Claim 1 or Claim 2, wherein the mixture comprises from 0.5 to 80 parts by weight of active ingredient, measured as the free acid, for each part by weight of antihistamine.

4.  A method according to Claim 3, wherein the mixture comprises from 6 to 40 parts by weight of active ingredient for each part by weight of antihistamine.

5.  A method according to Claim 4, wherein the mixture comprises from 1 to 10 parts by weight of active ingredient for each part by weight of antihistamine.

6. A method according to any one of the preceding claims, wherein the mixture comprises only one active ingredient and only one antihistamine.

7. A method of making a salt of 5-(2-hydroxypropoxy)-8-propyl-4H-1-benzopyran-2-carboxylic acid with those antihistamines having $H_2$ receptor antagonist properties which are sufficiently basic to form such a salt, using a metathetical process, or by reacting the free acid with the free antihistamine base.

## DOCUMENTS CONSIDERED TO BE RELEVANT

| Category | Citation of document with indication, where appropriate, of relevant passages | Relevant to claim |
|---|---|---|
| | DE - A - 2 810 974 (FISONS LTD) <br> * Pages 1,2; claims; page 4, page 9, last paragraph * <br> & GB - A - 1 582 362 <br> -- | 1-10 |
| A | DE - A - 1 917 123 (FISONS PHARMA-CEUTICALS LTD) <br> * Pages 38,39; claims 1-11 * <br> -- | 1-10 |
| AD | GB - A - 1 488 707 (FISONS LTD) <br> * Page 4, line 101 - page 5, line 71 * <br> ---- | 1-10 |

**CLASSIFICATION OF THE APPLICATION (Int Cl.³)**

```
A 61 K 45/06
        31/415
        31/505
        31/35//
(A 61 K 31/415
        31/35
        31/505
        31/35
        31/35
        31/34)
```

**TECHNICAL FIELDS SEARCHED (Int. Cl.³)**

```
A 61 K   31/35
         31/415
         31/505
C 07 D 311/24
```

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 26-08-1981 | BRINKMANN |

EPO Form 1503.1   06.78